# EUROPÄISCHE PATENTSCHRIFT

(11) **EP 3 041 438 B1**
(45) Veröffentlichungstag und Bekanntmachung des Hinweises auf die Patenterteilung: **29.11.2017**
(21) Anmeldenummer: 14736599.3
(22) Anmeldetag: 10.06.2014
(51) Int. Cl.: A61F 2/95

(54) **STENT-FALT- UND LADEVORRICHTUNG**
STENT, FOLDING, AND LOADING DEVICE
DISPOSITIF DE PLIAGE ET DE MISE EN PLACE D'UNE ENDOPROTHÈSE

(30) Priorität: 04.09.2013 DE 102013217614
(43) Veröffentlichungstag der Anmeldung: 13.07.2016
(73) Patentinhaber: Richard Wolf GmbH, 75438 Knittlingen (DE)
(72) Erfinder: FREY, Sebastian, 68753 Waghäusel (DE); MOSER, Florian, 75015 Bretten (DE)
(74) Vertreter: Patentanwälte Vollmann & Hemmer
(86) Internationale Anmeldenummer: PCT/DE2014/200254
(87) Internationale Veröffentlichungsnummer: WO 2015/032394

(56) Entgegenhaltungen:
- DE-A1- 10 004 979
- DE-B3- 10 249 927
- FR-A1- 2 902 641

## Beschreibung

Die Erfindung betrifft eine Stent-Falt- und Ladevorrichtung sowie ein Bronchoskopsystem mit einer solchen Stent-Falt- und Ladevorrichtung.

Es sind Stents bekannt, welche zum Einsatz in das Tracheal- und Bronchialsystem vorgesehen sind, um die Atemwege zu öffnen bzw. zu erweitern. Dies können Stents aus Metall und/oder Kunststoff sein. Um diese Stents in das Tracheal- und Bronchialsystem einbringen zu können, müssen diese vorher in ihrem Durchmesser verkleinert werden. Dies erfolgt üblicherweise durch Zusammenfalten. Der zusammengefaltete Stent wird dann in ein Bronchoskop eingesetzt und über das Bronchoskop im Bronchialsystem in Position gebracht. Problematisch bei diesen Systemen ist, dass der Stent blind positioniert und entfaltet werden muss. Darüber hinaus sind die bekannten Instrumentarien zum Zusammenfalten und Einbringen der Stents in den Tubus des Bronchoskops sehr kompliziert.

Aus DE 102 49 927 B3, FR 2 902 641 A1 sowie DE 100 04 979 A1 sind derartige Systeme zum Zusammenfalten und Einbringen von Stents bekannt.

Im Hinblick auf diese Problematik ist es Aufgabe der Erfindung eine Stent-Falt- und Ladevorrichtung sowie ein Bronchoskopsystem mit einer solchen Stent-Falt- und Ladevorrichtung bereit zu stellen, welche ein einfaches Falten des Stent und einen derartigen Einsatz in den Tubus des Bronchoskops ermöglicht, dass ein Einbringen des Stents in das Bronchialsystem unter visueller Kontrolle möglich wird.

Diese Aufgabe wird durch eine Stent-Falt- und Ladevorrichtung mit den in Anspruch 1 angegebenen Merkmalen sowie durch ein Bronchoskopsystem mit den in Anspruch 13 angegebenen Merkmalen gelöst.

Bevorzugte Ausführungsformen ergeben sich aus den nachfolgenden Unteransprüchen, der nachfolgenden Beschreibung sowie den beigefügten Figuren. Dabei ist zu verstehen, dass die nachfolgend beschriebenen Merkmale der bevorzugten Ausführungsformen einzeln oder in Kombination verwirklicht werden können.

Die erfindungsgemäße Stent-Falt- und Ladevorrichtung weist eine Hohlschiene auf, welche zum Falten und zur Aufnahme eines Stents ausgebildet ist. In einem Querschnitt quer zu ihrer Längsachse weist die Hohlschiene einen bogenförmigen d. h. im Wesentlichen U-förmigen Querschnitt auf. Besonders bevorzugt ist dieser Querschnitt kreisbogenförmig, insbesondere halbkreisförmig ausgebildet. Die Hohlschiene bildet so ein Halbrohr, welches an einer Längsseite geöffnet ist. Die so gebildete seitliche Öffnung der Hohlschiene ist einer Führungsschiene zugewandt, welche ein zweites wesentliches Element der erfindungsgemäßen Stent-Falt- und Ladevorrichtung bildet. Die Führungsschiene bildet einen sich in Längsrichtung der Führungsschiene erstreckenden Eingriffssteg, welcher der Öffnung der Hohlschiene zugewandt ist. Die Führungsschiene weist bevorzugt im Querschnitt quer zu ihrer Längsachse einen im Wesentlichen I- oder T-förmigen Querschnitt auf, welcher einen länglichen Schenkel als Eingriffssteg bildet, der sich auf die Öffnung der Hohlschiene zu erstreckt bzw. dieser zugewandt ist.

Die Hohlschiene ist an ihrem proximalen Ende schwenkbar mit der Führungsschiene verbunden, sodass die Hohlschiene relativ zu der Führungsschiene verschwenkt werden kann. Die Schwenkbewegung erfolgt dabei bevorzugt um eine Schwenkachse, welche sich quer, insbesondere normal zur Längsachse der Führungsschiene und zur Längsachse der Hohlschiene erstreckt. Dies ermöglicht, dass die Hohlschiene relativ zu der Führungsschiene so verschwenkt werden kann, dass die Hohlschiene und Führungsschiene relativ zueinander scherenförmig auseinander- und zusammengeklappt werden können. So kann die Hohlschiene relativ zu der Führungsschiene in eine erste geschlossene Position verschwenkt werden, in welcher sich die Längsachsen der Führungsschiene und der Hohlschiene parallel zueinander erstrecken, d. h. bevorzugt liegen die Hohlschiene und die Führungsschiene in dieser Position direkt nebeneinander, wobei sie sich parallel zueinander erstrecken und zwischen beiden ein Aufnahmeraum von in Längsrichtung im Wesentlichen konstantem Querschnitt gebildet wird. In diesen Aufnahmeraum kann ein Stent aufgenommen bzw. gefaltet werden.

Bevorzugt kann es sich bei der Verbindung zwischen Hohlschiene und Führungsschiene um ein Gelenk handeln. So kann die Hohlschiene z. B. um eine feste Schwenkachse relativ zu der Führungsschiene verschwenken. Die schwenkbare bzw. gelenkige Verbindung zwischen Holschiene und Führungsschiene kann jedoch auch auf andere Weise realisiert werden, beispielsweise durch einen geeigneten gelenkigen Eingriff oder durch eine elastische Verbindung, wie z. B. durch ein Federelement. Alternativ wäre es auch möglich, die Hohlschiene und/oder die Führungsschiene in zumindest ihrem proximalen Endbereich federnd bzw. elastisch verformbar auszubilden. Es ist keine feste, d. h. körperliche Schwenkachse erforderlich, vielmehr können die Hohlschiene und die Führungsschiene z. B. relativ zueinander um eine oder mehrere virtuelle Schwenkachsen verschwenken, welche sich bevorzugt in der oben genannten Richtung erstrecken. Die Führungsschiene und die Hohlschiene bewegen sich bzw. verschwenken bevorzugt in einer von ihren Längsachsen aufgespannten Ebene.

Wenn die Hohlschiene relativ von der Führungsschiene weg verschwenkt wird, d. h. beide auseinander geschwenkt werden, schneiden sich die Längsachsen der Hohlschiene und der Führungsschiene vorzugsweise in der Nähe oder in der Schwenkachse, welche das Gelenk bildet. D. h. in der geöffneten Position entfernen sich Führungsschiene und Hohlschiene in ihrer Erstreckungsrichtung ausgehend von der Schwenkachse, d. h. sie erstrecken sich ausgehend von der Schwenkachse V-förmig auseinander. In dieser Stellung kann ein Stent eingesetzt werden, welcher dann anschließend beim Zusammenklappen in das Innere der Hohlschiene hineinbewegt und in dieser gefaltet aufgenommen wird. Im geschlossenen Zustand kann die Stent-Falt- und Ladevorrichtung dann mit dem aufgenommenen und gefalteten Stent in das distale Ende eines Tubus eines Bronchoskops eingeschoben werden. Dazu ist die Außenkrümmung der Hohlschiene bevorzugt an den Innendurchmesser des Tubus so angepasst, dass beim Einsetzen die Außenseite der Hohlschiene am Innenumfang des Tubus zur Anlage kommt. Der gefaltete Stent kann durch Herausschieben aus dem Aufnahmeraum zwischen Führungsschiene und Hohlschiene in den Tubus eingebracht werden, um dann anschließend mittels des Tubus in das Bronchialsystem eingebracht zu werden.

Die Führungsschiene dient dazu, dass der Stent sich um die Führungsschiene herum faltet. D. h. bevorzugt wird der Stent von der Führungsschiene in einem Umfangsbereich in radialer Richtung eingedrückt, sodass dieser Umfangsbereich zu dem gegenüberliegenden Umfangsbereich des Stents bewegt wird. Beim Einbringen des so zusammengedrückten Stents in die Hohlschiene, wenn die Hohlschiene in die geschlossene Position bewegt wird, wird der Stent dabei um die Führungsschiene herum gefaltet, sodass er im Querschnitt die Form zweier ineinander liegender U-Bögen aufweist, welche an ihren Enden miteinander verbunden sind. In dieser gefalteten Position kann der Stent dann in den Tubus eines Bronchoskops eingeschoben werden, wobei im Bronchoskop der Raum, welcher von der Führungsschiene beim Falten eingenommen wird, dann von einem Endoskop- bzw. Optikschaft eingenommen werden kann. Dieses Falten ermöglicht so, den Stent in der Weise in einen Bronchoskoptubus einzubringen, dass später mit dem Bronchoskop die Applikation des Stents im Bronchialsystem unter visueller Kontrolle möglich ist.

Das Falten des Stents zum Einbringen in den Tubus eines Bronchoskops ist mit der erfindungsgemäßen Vorrichtung sehr einfach, da der Stent lediglich an die Führungsschiene angelegt wird und anschließend die Hohlschiene zu der Führungsschiene hin in die geschlossene Position verschwenkt wird, wobei der Stent in das Innere der Hohlschiene hineingedrückt und um die Führungsschiene herum gefaltet wird. Anschließend können dann die Führungsschiene und die Hohlschiene mit ihrem distalen Ende voran gemeinsam in das Innere eines Tubus eines Bronchoskops eingeschoben werden und der gefaltete Stent kann dann aus dem Aufnahmeraum zwischen Führungsschiene und Hohlschiene heraus in den Tubus eingeschoben werden.

Der von der Führungsschiene gebildete Eingriffssteg, welcher wie oben beschrieben, vorzugsweise I-förmig ausgebildet ist, erstreckt sich im Querschnitt weiter bevorzugt in Durchmesserrichtung der Hohlschiene, d. h. die längere Erstreckung des Querschnittes des Eingriffsabschnittes ist auf die Öffnung der Hohlschiene gerichtet und erstreckt sich radial bzw. in Durchmesserrichtung zur Längsachse der Hohlschiene.

Weiter bevorzugt greift der Eingriffssteg in der geschlossenen Position der Hohlschiene in die Öffnung der Hohlschiene ein. So kann der Eingriffsabschnitt den Stent beim Zusammenklappen von Hohlschiene und Führungsschiene in das Innere der Hohlschiene hineindrücken und dabei gleichzeitig zusammenfalten, sodass der Stent in einen Aufnahmeraum zwischen dem Innenumfang der Hohlschiene und den beiden entgegengesetzten sich in Längsrichtung erstreckenden Seitenflächen des Eingriffsabschnittes zu liegen kommt.

Weiter bevorzugt erstreckt sich die Hohlschiene in distaler Richtung über das distale Ende der Führungsschiene hinaus. So wird erreicht, dass die Hohlschiene den aufgenommen Stent weitgehend umgeben kann und beim Einschieben der Stent-Falt- und Ladevorrichtung in einen Tubus der Stent von der Hohlschiene überdeckt und geschützt wird. So wird das Einschieben in den Tubus erleichtert und dabei eine Beschädigung des Stents verhindert.

Gemäß einer weiteren bevorzugten Ausführungsform weist die erfindungsgemäße Stent-Falt- und Ladevorrichtung eine sich parallel zu der Führungsschiene erstreckende Aufnahmeschiene auf, welche ein distales freies Ende aufweist und derart ausgestaltet ist, dass ein Stent von diesem distalen Ende her auf die Aufnahmeschiene aufschiebbar ist. D. h. die Aufnahmeschiene ist ausgebildet, in das Innere des Stents einzugreifen und so den Stent relativ zu der Führungsschiene zu positionieren und zu halten. Vorzugsweise wird der Stent vom distalen Ende her zunächst auf die Aufnahmeschiene aufgeschoben und kommt seitlich der Führungsschiene zu liegen. Anschließend wird die Hohlschiene zu der Führungsschiene hin in die geschlossene Position verschwenkt, wobei der Stent mit der Führungsschiene in das Innere der Hohlschiene verschoben und dabei gefaltet wird. Die Aufnahmeschiene verbleibt dabei im Inneren des Stents und kommt mit diesem im Inneren der Hohlschiene zu liegen. D. h. die Aufnahmeschiene ist so angeordnet, dass sie im geschlossenen Zustand der Hohlschiene vorzugsweise im Inneren der Hohlschiene bzw. des von der Hohlschiene begrenzten Raumes zu liegen kommt.

Besonders bevorzugt wird die Aufnahmeschiene von zwei sich seitlich des Eingriffssteges der Führungsschiene erstreckenden Stäben gebildet, wobei sich die beiden Stäbe beabstandet von dem Eingriffssteg an entgegengesetzten Seiten des Eingriffssteges erstrecken. Das bedeutet an jeder der beiden sich in Längsrichtung erstreckenden Seitenflächen des Eingriffssteges ist beabstandet jeweils ein Stab gelegen, sodass der Eingriffssteg der Führungsschiene zwischen den beiden Stäben gelegen ist. Bevorzugt sind die Stäbe dabei symmetrisch zu dem Eingriffssteg bzw. der Führungsschiene angeordnet. Der Stent wird so auf die beiden Stäbe aufgeschoben, dass die Stäbe in das Innere des Stents eingreifen. Gleichzeitig wird der Stent in einem Außenumfangsbereich so eingedrückt, dass die Führungsschiene bzw. deren Eingriffssteg an der Außenseite des Stents zu liegen kommt, sodass der Stent um den Eingriffssteg herum bogenförmig gefaltet wird.

Der Außenabstand der Stäbe ist weiter bevorzugt kleiner gewählt als ein Innendurchmesser eines passenden aufzunehmenden Stents. D. h. die einander abgewandten Außenflächen der beiden Stäbe sind um ein Maß beabstandet, welches vorzugsweise kleiner als der Innendurchmesser des Stents ist. So kann der Stent auf die beiden Stäbe zunächst ohne größere Verformung des Stents aufgeschoben werden.

Die Ausgestaltung der Aufnahmeschiene in Form zweier Stäbe hat darüber hinaus den Vorteil, dass es auch möglich ist, einen Y-Stent, d. h. einen Stent, welcher eine Verzweigung aufweist, aufzunehmen. Ein solcher Stent kann derart aufgeschoben werden, dass jeweils ein Stab in einen der Zweige des Y-Stents eingreift, wobei der Stent mit seiner Verzweigung voran auf die distalen Enden der Stäbe aufgeschoben wird. Die beiden Stäbe treten dann durch die Zweige in den Bereich des Stents ein, welcher nur einen Durchgang aufweist. So kann auch ein solcher Y-Stent mit der erfindungsgemäßen Stent-Falt- und Ladevorrichtung gefaltet werden. Anschließend wird der so gefaltete Stent aus dem Aufnahmeraum zwischen Führungsschiene und Hohlschiene heraus in das distale Ende eines Bronchoskop-Tubus eingeschoben, wobei dieses Einschieben mit dem einlumigen Abschnitt des Y-Stents voran erfolgt, sodass die beiden Zweige des Y-Stents im Tubus dann distalwärts gerichtet sind, sodass beim Applizieren des Stents im Bronchialsystem dieser dann mit den beiden Zweigen voran in das Bronchialsystem eingebracht werden kann.

Weiter bevorzugt ist die Aufnahmeschiene mit ihrem distalen Ende elastisch relativ zu der Führungsschiene in einer Richtung quer zur Längsachse der Führungsschiene auslenkbar. So kann die Aufnahmeschiene zum Aufbringen des Stents am distalen Ende in einer Richtung quer zur Erstreckungsrichtung der Führungsschiene von dieser wegbewegt werden, sodass der Stent zunächst ohne Behinderung durch die Führungsschiene auf die Aufnahmeschiene aufgeschoben werden kann. Anschließend bewegt sich die Aufnahmeschiene zu der Führungsschiene zurück, wobei die Führungsschiene mit ihrem Eingriffssteg mit dem Außenumfang des Stents in Eingriff tritt und diesen dann in der oben beschriebenen Weise radial eindrückt und zusammenfaltet. Anstatt die Aufnahmeschiene elastisch auszubilden, kann diese Beweglichkeit auch durch eine Schwenkbarkeit einer starren Aufnahmeschiene relativ zu der Führungsschiene erreicht werden. Dazu könnten die Führungsschiene und Aufnahmeschiene ebenfalls im Bereich ihrer proximalen Enden schwenkbar aneinander angelenkt sein. Die Bewegbarkeit der Aufnahmeschiene erfolgt vorzugsweise in derselben Richtung bzw. derselben Ebene wie die Schwenkbewegung zwischen Hohlschiene und Führungsschiene, d. h. die Schwenkachsen erstrecken sich parallel zueinander. In dem Fall, dass die Aufnahmeschiene von zwei parallelen Stäben gebildet wird, sind diese beiden Stäbe vorzugsweise elastisch verformbar oder in der beschriebenen Weise schwenk- oder auslenkbar ausgebildet. Die beiden Stäbe werden dabei parallel zueinander jeweils in einer Ebene, welche sich parallel zu dem Eingriffssteg der Führungsschiene erstreckt, von der Führungsschiene weg bewegt oder geschwenkt. Gleichzeitig kann auch eine Elastizität der Stäbe in einer Querrichtung zu dieser Schwenkbewegung gegeben sein, welche ein Aufschieben des Stents auf die Stäbe erleichtert.

Zweckmäßigerweise erstreckt sich die Aufnahmeschiene mit ihrem distalen Ende distalwärts über das distale Ende der Führungsschiene hinaus. D. h. in distaler Richtung ist die Aufnahmeschiene länger als die Führungsschiene ausgebildet. Dies ermöglicht es, dass der Stent vom distalen Ende her auf die Aufnahmeschiene, insbesondere deren vorangehend beschriebenen zwei Stäbe aufgeschoben werden kann, ohne gleich zu Beginn mit der Führungsschiene in Kontakt zu kommen.

Weiter bevorzugt ist die Führungsschiene an ihrem distalen Ende angeschrägt ausgebildet. Dabei erstreckt sich eine Stirnkante der Führungsschiene bzw. deren Eingriffssteges, welche der Öffnung der Hohlschiene zugewandt ist, am distalen Ende gewinkelt von der Hohlschiene weg. So kann der Stent, wenn er auf die Aufnahmeschiene aufgeschoben wird, über die Schräge auf die Führungsschiene aufgeschoben werden, wobei der Eingriffssteg der Führungsschiene dann den Stent wie beschrieben an einen Umfangsbereich zusammendrückt, sodass dieser in der beschriebenen Weise gefaltet wird.

Der Innenquerschnitt der Hohlschiene ist vorzugsweise kleiner ausgebildet als der Außenquerschnitt eines passenden aufzunehmenden Stents. Dadurch wird es möglich, den Stent so zusammenzufalten, dass er innerhalb der Hohlschiene einen kleineren Außenquerschnitt einnimmt als er im entfalteten Zustand hat.

Gemäß einer besonders bevorzugten Ausführungsform der Erfindung weist die Stent-Falt- und Ladevorrichtung darüber hinaus einen Stößel auf, welcher axial entlang der Führungsschiene bzw. in Richtung deren Längsachse vorschiebbar ist. Ein solcher Stößel dient dazu, den gefalteten Stent aus der Stent-Falt- und Ladevorrichtung, d. h. dem zwischen Führungsschiene und Hohlschiene gebildeten Aufnahmeraum in distaler Richtung herauszuschieben, um ihn so in das Inneren eines Tubus eines Bronchoskops einschieben zu können. Der Stößel ist dazu bevorzugt gleitend auf der Führungsschiene und/oder der Aufnahmeschiene geführt. D. h. der Stößel weist zweckmäßigerweise im Querschnitt passende Ausnehmungen auf, durch welche sich die Führungsschiene und die Aufnahmeschiene in Richtung ihrer Längsachsen hindurch erstrecken. Die Ausnehmungen weisen zweckmäßigerweise einen Querschnitt auf, welcher dem Querschnitt der Führungsschiene bzw. der Aufnahmeschiene entspricht. In dem Fall, dass die Aufnahmeschiene von zwei Stäben gebildet wird, weist der Stößel bevorzugt zwei voneinander beanstandete, sich in Längsrichtung, d. h. vom proximalen zum distalen Ende durch den Stößel hindurch erstreckende Löcher auf, durch welche sich die Stäbe hindurch erstrecken. Um die Schwenk- bzw. elastische Verformbarkeit der Aufnahmeschiene, insbesondere der Stäbe zu ermöglichen, können diese Löcher dabei einen länglichen oder ovalen Querschnitt aufweisen, welcher eine Beweglichkeit der Aufnahmeschiene in den Löchern ermöglicht.

Weiter bevorzugt weist der Stößel an seiner proximalen Seite zumindest eine Vorschubstange auf, welche sich durch eine Führung erstreckt, welche am proximalen Ende der Führungsschiene angeordnet ist. Über diese Vorschubstange kann der Stößel in distaler Richtung vorgeschoben werden, um den gefalteten Stent aus dem Aufnahmeraum zwischen Hohlschiene und Führungsschiene in distaler Richtung heraus und in das Innere eines Tubus eines Bronchoskops zu schieben. An ihrem proximalen Ende weist die zumindest eine Vorschubstange dabei vorzugsweise ein Griffstück auf, welches eine vergrößerte Oberfläche aufweist und so eine gute Handhabung bietet. Die Länge der Vorschubstange ist bevorzugt so gewählt, dass sie im Wesentlichen der Länge der Führungsschiene entspricht, sodass mit Hilfe der Vorschubstange der Stößel bis zum distalen Ende der Führungsschiene verschoben werden kann. Dabei ist die Länge jedoch so gewählt, dass der Stößel nicht vom distalen Ende der Führungsschiene heruntergeschoben werden kann, d. h. die Länge der Vorschubstange ist so gewählt, dass sie die Bewegung des Stößels in distaler Richtung durch Ihre Länge begrenzt. Dabei kommt das Griffstück am proximalen Ende der Betätigungsstange vorzugsweise proximalseitig an der Führung der Vorschubstange zur Anlage. Die Führung ist vorzugsweise in einen proximalen Endabschnitt integriert, in welchem ebenfalls die Führungsschiene und die Aufnahmeschiene gelagert sind. Der proximale Endabschnitt kann darüber hinaus so erweitert ausgebildet sein, dass er einen Griffabschnitt bildet, an welchen die Vorrichtung ergriffen und beim Einschieben des Stents in ein Bronchoskop gehalten werden kann. dabei kann vorzugsweise der Griffabschnitt mit den Fingern gehalten werden, während gleichzeitig der Daumen derselben Hand gegen einen Betätigungsabschnitt bzw. Griffstück der Vorschubstange drückt, um den Stent aus der Vorrichtung heraus in den Tubus des Bronchoskops zu verschieben. So kann die Stent-Falt- und Ladevorrichtung einhändig gehandhabt werden, um den Stent in das Bronchoskop einzubringen. Mit der anderen Hand kann dabei das Bronchoskop gehalten werden.

Besonders bevorzugt sind zwei zueinander parallele Vorschubstangen vorgesehen. Diese ermöglichen einen verdrehsicheren Vorschub des Stößels. Darüber hinaus können sich diese beiden Vorschubstangen seitlich entlang der Führungsschiene erstrecken. Dabei wird eine gleichmäßige symmetrische Kraftübertragung auf den Stößel gewährleistet.

Gegenstand der Erfindung ist ferner ein Bronchoskopsystem bestehend aus einem Bronchoskop und einer Stent-Falt- und Ladevorrichtung gemäß der obigen Beschreibung, welche dazu vorgesehen ist, einen Stent in dieses Bronchoskop einbringen zu können. Das Bronchoskop weist in bekannter Weise einen Bronchoskop-Tubus und ein darin angeordnetes Endoskop mit einer Optik auf. Das Endoskop weist dazu einen Endoskop- bzw. Optikschaft auf, welcher sich im Inneren des Tubus in Längsrichtung erstreckt. Das Endoskop ist vorzugsweise entnehmbar in dem Tubus gehalten.

Die Stent-Falt- und Ladevorrichtung des erfindungsgemäßen Bronchoskopsystems ist in ihrer Dimensionierung auf das zugehörige Bronchoskop und insbesondere auf die Größe des Bronchoskop-Tubus abgestimmt. So ist der Außenquerschnitt, welchen die Hohlschiene und die Führungsschiene in ihrer geschlossenen Position begrenzen, derart kleiner ausgestaltet als der Innenquerschnitt des Bronchoskop-Tubus, dass die Stent-Falt- und Ladevorrichtung in der geschlossenen Position der Hohlschiene mit ihrem distalen Ende in eine distale Öffnung des Bronchoskop-Tubus einführbar ist. D. h. Führungsschiene und Hohlschiene können mit ihren distalen Endebereichen in die Öffnung des Bronchoskop-Tubus eingesetzt werden, sodass dann aus dem Inneren des von Führungsschiene und Hohlschiene begrenzten Aufnahmeraumes der gefaltete Stent vorzugsweise mit Hilfe des oben beschriebenen Stößels in den Innenraum des Bronchoskop-Tubus von dessen distalen Ende her eingeschoben werden kann. Die Hohlschiene hat wie oben beschrieben vorzugsweise eine Querschnittskontur, welche der Innenkontur des Tubus in dessen Querschnitt entspricht.

Bevorzugt ist das Endoskop in dem Bronchoskop-Tubus exzentrisch angeordnet, d. h. der Schaft des Endoskops erstreckt sich im Bronchoskop-Tubus nicht zentral sondern aus der Mitte radial nach außen versetzt parallel zur Längsachse des Tubus von dessen proximalen zum distalen Ende. Dabei ist das Endoskop so exzentrisch angeordnet, dass dann, wenn die Stent-Falt- und Ladevorrichtung in die distale Öffnung des Bronchoskop-Tubus eingeführt ist, die Führungsschiene mit ihrem distalen Ende dem distalen Ende des Endoskops gegenüberliegt oder sich radial weiter einwärts gelegen, aber vorzugsweise in derselben Durchmesserebene, parallel zu dem Endoskop erstreckt. So kann der gefaltete Stent von der Führungsschiene in das Innere des Bronchoskops eingeschoben werden, wobei das Endoskop den Raum einnimmt, welcher von dem gefalteten Stent nicht eingenommen wird und zuvor von der Führungsschiene eingenommen bzw. durch Einfalten des Stents geschaffen wurde. D. h. der gefaltete Stent wird von der Führungsschiene über den Schaft des Endoskops geschoben. So kann der gefaltete Stent in dem Bronchoskop-Tubus seitlich des Endoskops liegen, sodass dieses sich parallel zu dem gefalteten Stent bis zum distalen Ende des Bronchoskop-Tubus erstrecken kann. Auf diese Weise kann mit dem Bronchoskop der Stent unter optischer Kontrolle im Bronchialsystem platziert werden. Es ist so nicht erforderlich, zum Applizieren des Stents das Endoskop aus dem Bronchoskop-Tubus zu entnehmen. Außerdem beeinträchtigt der gefaltete Stent im Inneren des Bronchoskop-Tubus nicht das Sichtfeld des Endoskops. Zudem kann eine erforderliche Röntgenexposition so gering wie möglich gehalten werden.

Besonders bevorzugt weist das erfindungsgemäße Bronchoskopsystem zusätzlich zumindest einen angepassten Stent auf, dessen Außendurchmesser größer als der Innendurchmesser des Bronchoskop-Tubus und der Querschnitt der Hohlschiene der Stent-Falt- und Ladevorrichtung ist und welcher gefaltet in der Hohlschiene aufnehmbar ist. Der Stent ist in seiner Größe so gewählt, dass er die gewünschte Funktion im Inneren des Bronchialsystems übernehmen kann. Dabei ist der Stent derart elastisch oder faltbar ausgebildet, dass er auf eine Größe zusammengefaltet werden kann, dass er im Inneren des Bronchoskop-Tubus aufgenommen werden kann. Die Stent-Falt- und Ladevorrichtung ist in ihrer Größe so angepasst, dass sie den Stent auf das gewünschte Maß in dem Aufnahmeraum zwischen Führungsschiene und Hohlschiene zusammenfalten kann, sodass der gefaltete Stent dann in den Innenraum des Bronchoskop-Tubus einschiebbar ist.

Das Bronchoskopsystem mit der Stent-Falt- und Ladevorrichtung ermöglichen eine neuartige Platzierung eines Stents im Inneren des Bronchoskop-Tubus und einen neuartigen dafür geeigneten Faltvorgang, d. h. ein neuartiges Faltverfahren, welches ebenfalls Gegenstand dieser Erfindung ist und sich aus der obigen Beschreibung des Stent-Falt- und Ladesystems ergibt.

Nachfolgend wird die Erfindung beispielhaft anhand der beigefügten Figuren beschrieben. In diesen zeigt:
- Fig. 1: eine Seitenansicht einer erfindungsgemäßen Stent-Falt- und Ladevorrichtung in einer geöffneten Position,
- Fig. 2: eine Draufsicht auf das distale Ende der Stent-Falt- und Ladevorrichtung gemäß Fig. 1 in der geschlossenen Position,
- Fig. 3: eine perspektivische Ansicht der Stent-Falt- und Ladevorrichtung gemäß Fig. 1 mit einem aufzunehmen Stent,
- Fig. 4: vergrößert das distale Ende der Stent-Falt- und Ladevorrichtung gemäß Fig. 3 mit dem aufzunehmenden Stent,
- Fig. 5: das distale Ende der Stent-Falt- und Ladevorrichtung gemäß Fig. 1 und 4 mit aufgesetztem Stent,
- Fig. 6: die Stent-Falt- und Ladevorrichtung gemäß Fig. 1 - 5 im geschlossenen Zustand,
- Fig. 7: die Stent-Falt- und Ladevorrichtung gemäß Fig. 1 - 6 mit aufgenommenen Stent in geschlossenem Zustand und einem zugehörigen Bronchoskop,
- Fig. 8: das Bronchoskop mit der Stent-Falt- und Ladevorrichtung gemäß Fig. 4, wobei die Vorrichtung in das Bronchoskop eingesetzt ist und

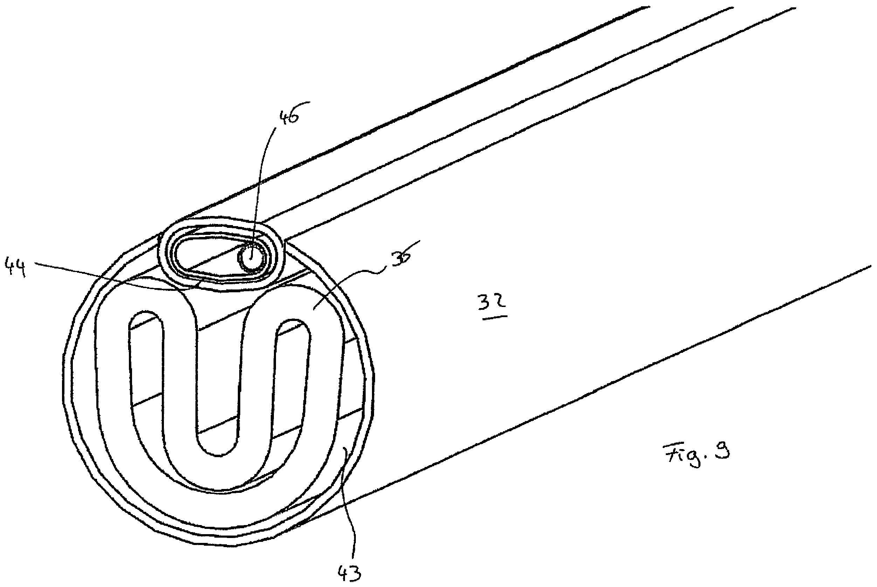

- Fig. 9: einen Schnitt im distalen Bereich des Bronchoskoptubus gemäß Fig. 7 und 8 mit eingesetztem Stent.

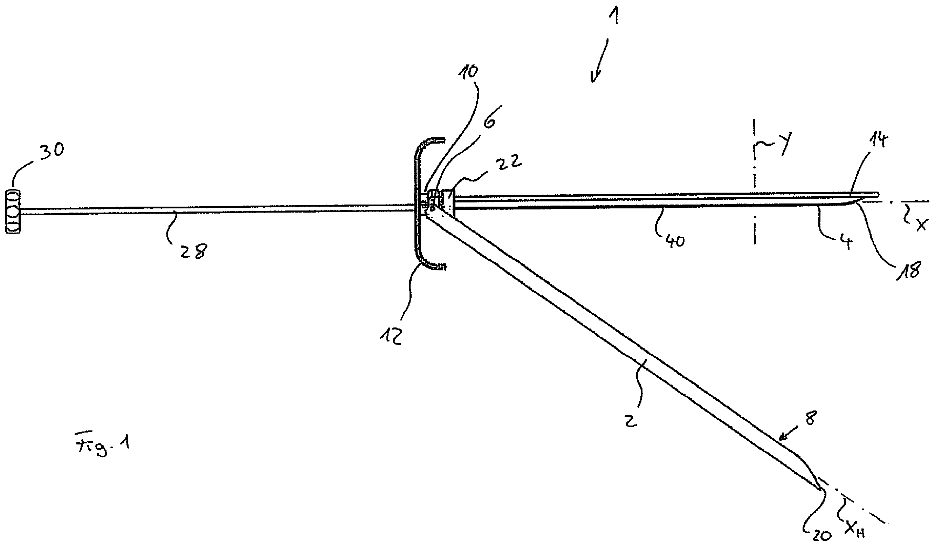

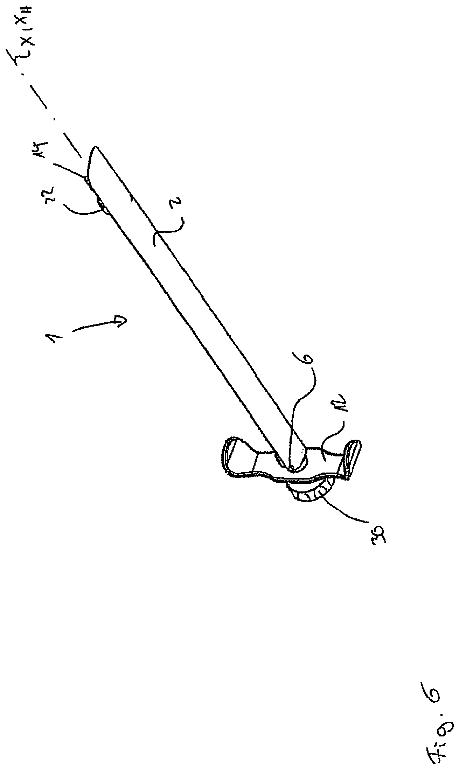

Die in den Figuren gezeigte Stent-Falt- und Ladevorrichtung 1 weist als wesentliche Elemente eine als Halbrohr ausgebildete Hohlschiene 2 sowie eine Führungsschiene 4 auf. An ihren proximalen Enden sind die Hohlschiene 2 und die Führungsschiene 4 über eine Schwenkachse 6 gelenkig miteinander verbunden. Die Führungsschiene 4 ist starr angeordnet und erstreckt sich entlang der Längsachse X des Instrumentes bzw. der Stent-Falt- und Ladevorrichtung. Die Hohlschiene 2 ist mit ihrer Längsachse X_{H} um die Schwenkachse 6 relativ zu der Führungsschiene 4 scherenförmig verschwenkbar, wobei Fig. 1 eine geöffnete Position und Fig. 6 eine geschlossene Position zeigt. In der geschlossenen Position erstrecken sich die Längsachse X der Führungsschiene 4 und die Längsachse X_{H} der Hohlschiene 2 parallel bzw. deckungsgleich zueinander. Die Schwenkachse 6 erstreckt sich normal bzw. rechtwinklig zu den Längsachsen X und X_{H}.

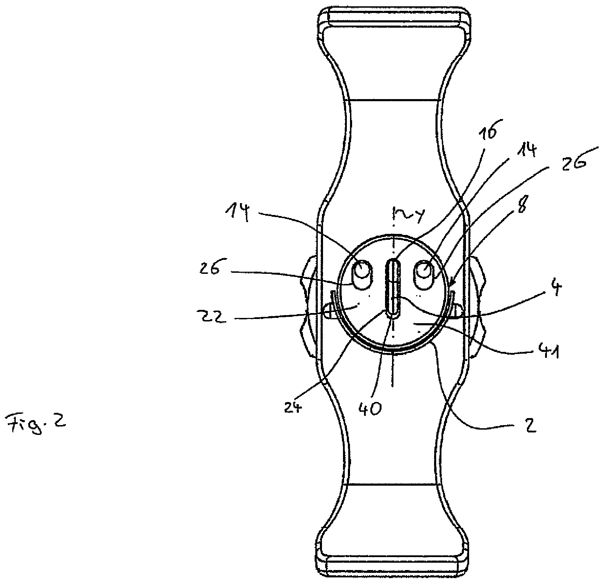

Die Hohlschiene 2 weist, wie in der distalseitigen Draufsicht in Fig. 2 zu erkennen ist, einen halbkreisförmigen Querschnitt mit einer sich in Längsrichtung erstreckenden seitlichen Öffnung 8 auf. Die Führungsschiene 4 weist, wie ebenfalls in der Draufsicht in Fig. 2 zu sehen ist, einen I-förmigen Querschnitt auf. Die Führungsschiene 4 erstreckt sich dabei im Querschnitt mit ihrer längeren Querausdehnung Y in Durchmesserrichtung bzw. radialer Richtung bezogen auf die Längsachse X_{H} der Hohlschiene 2. Die Führungsschiene 4 bildet dabei einen Eingriffssteg, welcher in der in Fig. 2 gezeigten geschlossenen Position mit seinem der Hohlschiene 2 zugewandten Abschnitt in das Innere der Hohlschiene 2 eingreift. D. h. die Führungsschiene 4 greift in die Öffnung 8 der Hohlschiene 2, welche sich an einer Längsseite der Hohlschiene 2 parallel zu der Längsachse X_{H} erstreckt, ein.

Die Schwenkachse 6 ist am proximalen Ende der Führungsschiene 4 angebracht. Die Führungsschiene 4 ist mit ihrem proximalen Ende dabei in einem proximalen Endabschnitt bzw. Endkörper 10 angebracht, welcher eine Handhabe 12 bzw. einen Griffabschnitt 12 trägt, an welchen das Instrument gehalten werden kann.

Seitlich der Führungsschiene 4 erstrecken sich zwei Stäbe 14, welche eine Aufnahmeschiene bilden. Die Stäbe 14 erstrecken sich seitlich der Führungsschiene parallel und symmetrisch zu deren Längsachse X und beabstandet zu den entgegengesetzten Seitenflächen der Führungsschiene 4. Die Anordnung der Stäbe 14 ist dabei im Querschnitt ebenfalls symmetrisch zu der Achse Y der längeren Querausdehnung der Führungsschiene 4. Die Stäbe 14 liegen dabei in einer Ebene, welche sich im Bereich einer der Hohlschiene 2 abgewandten Seitenkante 16 der Führungsschiene 4 erstreckt. D. h. die Führungsschiene 4 steht mit der größten Querausdehnung in Richtung der Achse Y der Hohlschiene 2 zugewandt gegenüber den Stäben 14 vor und bildet so den Eingriffsabschnitt. Die Stäbe 14 sind ebenfalls in dem proximalen Endkörper 10 festgelegt. Die Stäbe 14 weisen eine gewisse Elastizität auf, welche eine elastische Auslenkung in Richtung der Achse Y, welche sich quer zur Längsachse X und der Schwenkachse 6 erstreckt, ermöglicht. Die distalen Enden der Stäbe 14 stehen über das distale Ende 18 der Führungsschiene 4 vor. In der in Fig. 6 gezeigten geschlossenen Position steht darüber hinaus das distale Ende 20 der Hohlschiene 2 über das distale Ende 18 der Führungsschiene 4 sowie über die distalen Enden der Stäbe 14 vor.

Ferner weist die Stent-Falt- und Ladevorrichtung 1, welche in den Figuren gezeigt ist, einen Stößel 22 auf, welcher axial beweglich in Richtung der Längsachse X auf der Führungsschiene 4 geführt ist. Der Stößel 22 ist scheibenförmig ausgebildet und weist sich in Richtung der Längsachse X erstreckende Durchgangslöcher 24, 26 auf. Dabei ist ein zentrales Durchgangsloch 24 in seinem Querschnitt dem Querschnitt der Führungsschiene 4 angepasst. D. h. das Durchgangsloch 24 weist eine geringfügig größere Querschnittsform als die Führungsschiene 4 auf, sodass der Stößel 22 drehfest in axialer Richtung X auf der Führungsschiene 4 verschiebbar geführt ist. Seitlich des Durchgangsloches 24 sind symmetrisch zur Mittelachse Y des Durchgangsloches 24 zwei Durchgangslöcher 26 in dem Stößel 22 ausgebildet, durch welche sich die Stäbe 14 erstrecken. Die Durchgangslöcher 26 weisen einen länglichen bzw. ovalen Querschnitt auf, welcher parallel zur Achse Y eine größere Länge als der Durchmesser der Stäbe 14 aufweist. Dies ermöglicht die elastische Auslenkung der Stäbe 14 in Richtung der Achse Y auch bei aufgeschobenem Stößel 22.

An der proximalen Seite des Stößels 22 sind zwei Vorschubstangen 28 angebracht, welche längsverschieblich in Richtung der Längsachse X durch den Endkörper 10 hindurchgeführt sind. An der dem Stößel 22 abgewandten proximalen Seite des Endkörpers 10 ist am proximalen Ende der Vorschubstangen 28 ein Griffstück 30 befestigt, über welches die Betätigungsstangen 28 und damit der Stößel 22 in axialer Richtung X auf der Führungsschiene 4 verschoben werden kann. Die Vorschubstangen 28 sind so lang ausgebildet, dass der Stößel 22 sich bis zum distalen Ende der Führungsschiene 4, aber nicht über dieses hinaus verschieben lässt. Um den Vorschubweg zu beschränken, schlägt dazu das Griffstück 30 an der proximalen Seite der Handhabe 12 bzw. des Endkörpers 10 an. D. h. die Länge der Vorschubstangen 28 entspricht im Wesentlichen der Länge der Führungsschiene 4. Bei der Handhabung kann das Instrument mit einer Hand so ergriffen werden, dass die Finger die Handhabe 12 ergreifen, während der Daumen auf das proximale Ende des Griffstückes 30 drückt, um den Stößel 22 über die Vorschubstangen 28 distalwärts vorschieben zu können, wie es nachfolgend beschrieben wird.

Der Außendurchmesser der Hohlschiene 2 ist an den Innendurchmesser eines Bronchoskop-Tubus 32 eines zugehörigen Bronchoskop 34 angepasst. Die in den Figuren 1 - 6 gezeigte Stent-Falt- und Ladevorrichtung 1 bildet somit gemeinsam mit dem Bronchoskop 34 ein Bronchoskopsystem. Mittels der Stent-Falt- und Ladevorrichtung 1 kann ein Stent 36 so gefaltet werden, dass er vom distalen Ende 38 her in das Innere des Bronchoskop-Tubus 32 eingebracht werden kann. Das Falten des Stents 36 erfolgt in der nachfolgend beschriebenen Weise.

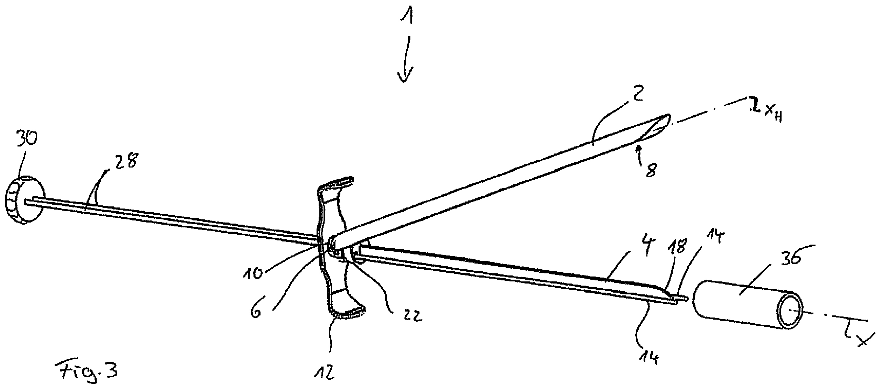

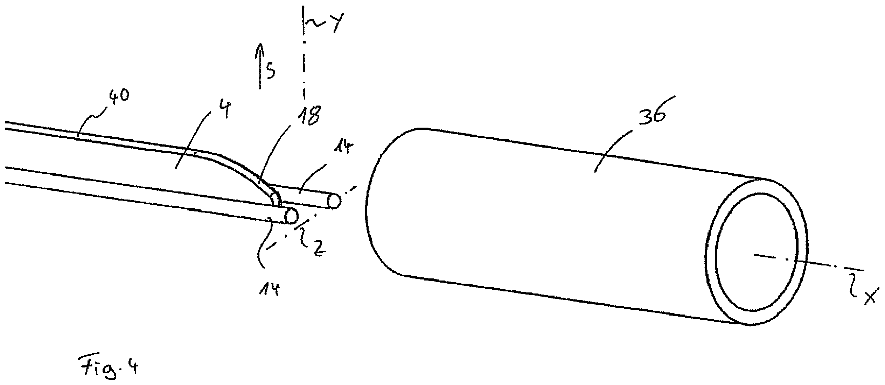

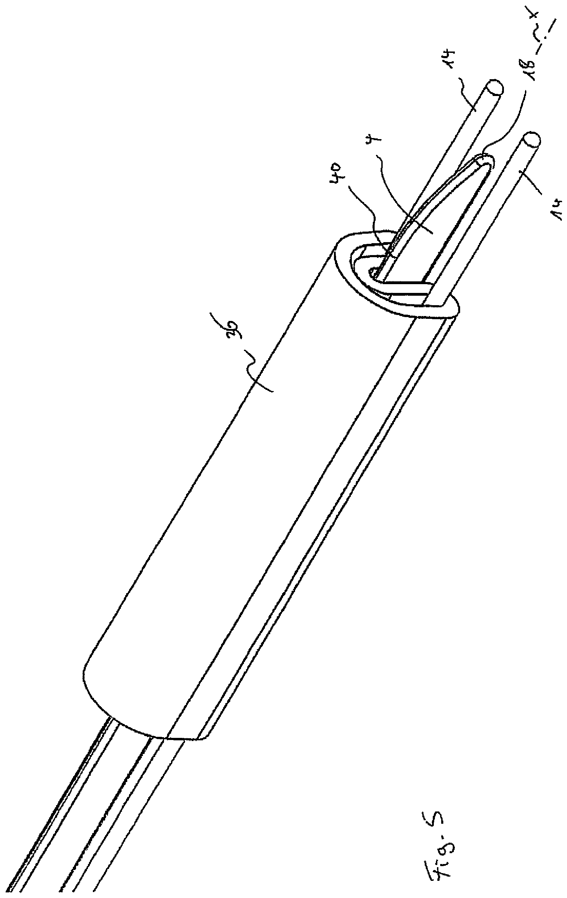

Wie in Fig. 3 und 4 zu erkennen ist, hat der Stent 36 in seiner entfalteten Lage in bekannter Weise einen kreisförmigen Querschnitt, d. h. er ist insgesamt rohrförmig ausgebildet. Der Stent 36 hat dabei einen Durchmesser, welcher größer ist als der Innendurchmesser des Bronchoskop-Tubus 32, sodass es erforderlich ist, den Stent 36 zum Einbringen in den Bronchoskop-Tubus 32 zusammenzufalten. Dazu wird der Stent 36 zuerst in Richtung der Längsachse X auf die Stäbe 14, welche eine Aufnahmeschiene bilden, aufgeschoben. Dabei treten die Stäbe 14 in das Innere des Stents 36 ein. Da die Außenseiten der Stäbe 14 in Richtung der Achse Z, welche sich normal zu den Seitenflächen der Führungsschiene 4 erstreckt, um ein Maß beabstandet sind, welches kleiner ist als der Innendurchmesser des Stents 36, können die Stäbe 14 ohne wesentliche Verformung des Stents 36 in dessen Innenraum eingreifen. Wenn der Stent 36 in distaler Richtung weiter auf die Stäbe 14 aufgeschoben wird, tritt er mit dem distalen Ende 18 der Führungsschiene 4 in Kontakt. Die Stäbe 14 werden aufgrund ihrer Elastizität dabei zunächst in Richtung des Pfeiles S in Fig. 4, d. h. parallel zur Achse Y ausgelenkt, sodass die Stäbe 14 von der Führungsschiene 4 in Richtung der Achse Y im Bereich des distalen Endes beabstandet werden. D. h. die distalen Enden der Stäbe 14 liegen dann näher zu der Hohlschiene 2 als die der Hohlschiene 2 zugewandte Längskante 40 der Führungsschiene 4. Die Führungsschiene 4 ist an ihrem distalen Ende 18 darüber hinaus angeschrägt ausgebildet, d. h. die Längskante 40 nähert sich am distalen Ende 18 der entgegengesetzten Seiten- bzw. Längskante 16 der Führungsschiene 4 an. So wird es begünstigt, dass der Stent 36 beim weiteren Vorschub so auf die Führungsschiene 18 aufgeschoben wird, dass die Längskante 40 der Führungsschiene 4 am Außenumfang des Stents 36 zu liegen kommt. Wenn die Stäbe 14 dann wieder in ihre gezeigte Ruhelage zurückbewegt werden, wird dabei der Stent 36, wie in Fig. 5 gezeigt, gefaltet.

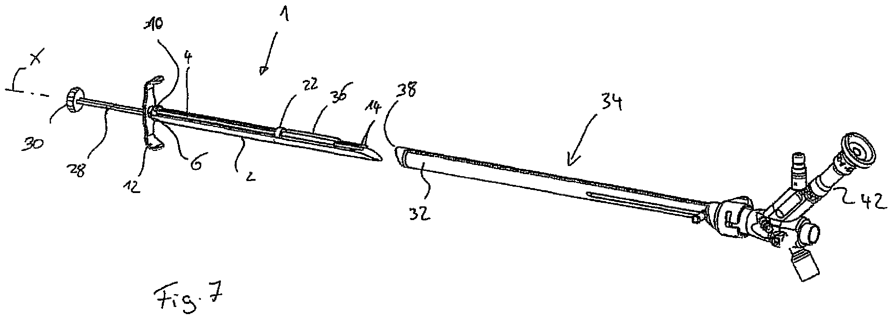

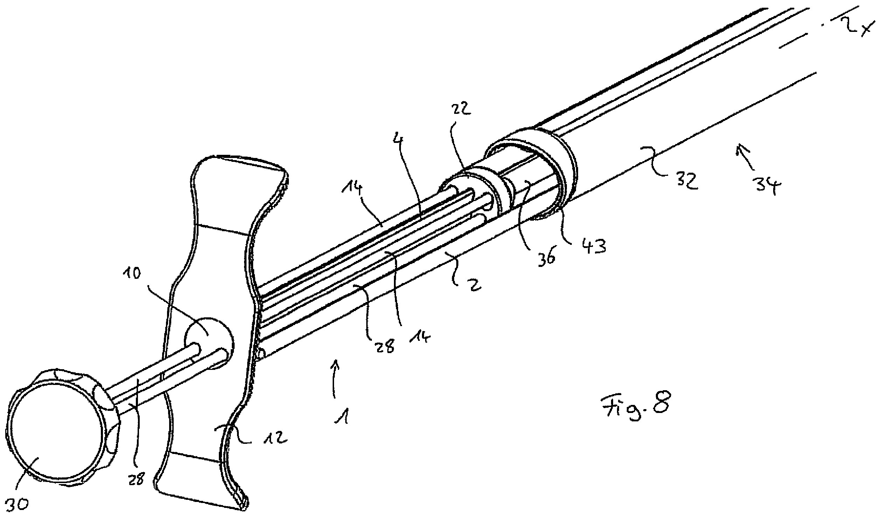

Die Führungsschiene 4, welche einen Eingriffssteg bildet, drückt einen Umfangsbereich des Stents 36 radial nach innen, sodass dieser Umfangsbereich im Wesentlichen an der Innenseite des gegenüberliegenden Umfangsbereiches zu liegen kommt. D. h. der Stent wird um die Führungsschiene 4 herumgefaltet, sodass er im Querschnitt die Form von zwei übereinanderliegenden U-förmigen Bögen einnimmt. In diesem Zustand wird dann die Hohlschiene 2 in Richtung der Führungsschiene 4 geklappt, sodass der gefaltete Stent 36 im Innenraum der Hohlschiene 2 zu liegen kommt. Der gefaltete Stent 36 liegt dann in einem Aufnahmeraum 41, welcher zwischen der Führungsschiene 4 und dem Innenumfang der Hohlschiene 2 gebildet wird. Dieser Zustand ist in den Figuren 7 und 8 gezeigt. Fig. 6 zeigt die geschlossene Position der Hohlschiene 2 ohne aufgenommen Stent.

Der Stößel 22 wird durch Druck auf das Griffstück 30 so weit distalwärts verschoben, bis er an der proximalen Seite des Stents 36 zur Anlage kommt. Anschließend wird die Stent-Falt- und Ladevorrichtung mit ihrem distalen Ende voran in die distale Öffnung 43 des Bronchoskop-Tubus 32 eingeschoben. Da die Hohlschiene 2 sich in distaler Richtung am längsten erstreckt, tritt die Hohlschiene 2 zur Führung in den Innenumfang des Bronchoskop-Tubus 32 ein. Wenn die Hohlschiene 2 mit der Führungsschiene 4 und dem aufgenommenen Stent 36 so in dem Bronchoskop-Tubus 32 eingesetzt ist, wird durch Druck auf das Griffstück 30 über die Vorschubstangen 28 der Stößel 22 weiter distalwärts bewegt, wodurch der Stent 36 in das Innere des Bronchoskop-Tubus 32 geschoben wird. Wenn der Stent 36 vollständig im Inneren des Bronchoskop-Tubus 32 angeordnet ist, kann die Stent-Falt- und Ladevorrichtung 1 wieder aus dem Bronchoskop-Tubus 32 herausgezogen werden.

Das Bronchoskop 34 weist ein Endoskop 42 auf, welches vom proximalen Ende her in dem Bronchoskop-Tubus 32 derart eingesetzt ist, dass sich ein Endoskopschaft 44 mit einer Optik 46 im Inneren des Bronchoskop-Tubus 32 entlang dessen Umfangswandung erstreckt. D. h. das Endoskop 42 ist so ausgebildet und angeordnet, dass der Endoskopschaft 44 exzentrisch im Inneren des Bronchoskop-Tubus 32 gelegen ist. Dabei erstreckt sich der Endoskopschaft 44 in einem Freiraum, welcher von dem gefalteten Stent 36 gelassen wird. Dieser Freiraum wird durch die Einbuchtung der Umfangswandung des Stents 36 geschaffen, welche von der Führungsschiene 4 bewirkt wird. Beim Einsetzen des Stents 36 in den Bronchoskop-Tubus 32 liegt daher die Führungsschiene 4 radial nach innen verschoben parallel zu dem Endoskopschaft 44, sodass die Einbuchtung des Stents 36 im Bereich des Endoskopschafts 44 zu liegen kommt. Diese Ausgestaltung ermöglicht es, dass sich der Endoskopschaft 44 mit der Optik 46 parallel zu dem Stent 36 erstrecken kann, sodass der Stent 36 das Blickfeld der Optik 46 nicht beeinträchtigt und das Einbringen des Stents 36 mit Hilfe des Bronchoskops unter visueller Kontrolle möglich ist.

### Bezugszeichenliste

- 1: Stent-Falt- und Ladevorrichtung
- 2: Hohlschiene
- 4: Führungsschiene
- 6: Schwenkachse
- 8: Öffnung
- 10: Endkörper
- 12: Handhabe
- 14: Stäbe
- 16: Seitenkante bzw. Längskante
- 18: distales Ende
- 20: distales Ende
- 22: Stößel
- 24, 26: Durchgangslöcher
- 28: Vorschubstangen
- 30: Griffstück
- 32: Bronchoskop-Tubus
- 34: Bronchoskop
- 36: Stent
- 38: distales Ende
- 40: Längskante
- 41: Aufnahmeraum
- 42: Endoskop
- 43: Öffnung
- 44: Endoskopschaft
- 46: Optik
- X: Instrumentenachse, Längsachse der Führungsschiene 4
- X_{H}: Längsachse der Hohlschiene 2
- Y: Achse der längeren Querausdehnung der Führungsschiene 4
- Z: Achse normal zu den Achsen Y und X
- S: Bewegungsrichtung der Stäbe 14

## Patentansprüche

1. Stent-Falt- und Ladevorrichtung (1) **gekennzeichnet durch**
eine Hohlschiene (2), welche einen bogenförmigen Querschnitt aufweist, sowie einer Führungsschiene (4), welche einen der Öffnung (8) der Hohlschiene (2) zugewandten, sich in Längsrichtung (X) der Führungsschiene (4) erstreckenden Eingriffssteg bildet, wobei
die Hohlschiene (2) an ihrem proximalen Ende schwenkbar mit einem proximalen Ende der Führungsschiene verbunden ist,
und
die Hohlschiene (2) relativ zu der Führungsschiene (4) zwischen einer geschlossen Position, in welcher die Längsachsen (X, X_{H}) von Führungsschiene (4) und Hohlschiene (2) parallel verlaufen, und einer geöffneten Position, in welcher die Längsachsen (X, X_{H}) der Führungsschiene (4) und der Hohlschiene (2) gewinkelt zueinander verlaufen, verschwenkbar ist.

2. Stent-Falt- und Ladevorrichtung (1) nach Anspruch 1, **dadurch gekennzeichnet, dass** die Hohlschiene (2) an ihrem proximalen Ende um eine Schwenkachse (6) relativ zu der Führungsschiene (4) verschwenkbar ist, wobei sich die Schwenkachse (6) quer zur Längsachse (X) der Führungsschiene (4) und zur Längsachse (X_{H}) der Hohlschiene (2) erstreckt.

3. Stent-Falt- und Ladevorrichtung nach Anspruch 1 oder 2, **dadurch gekennzeichnet, dass** der Eingriffssteg (4) sich im Querschnitt in Durchmesserrichtung der Hohlschiene (2) erstreckt.

4. Stent-Falt- und Ladevorrichtung nach einem der vorangehenden Ansprüche, **dadurch gekennzeichnet, dass** der Eingriffssteg (4) in der geschlossenen Position in die Öffnung (8) der Hohlschiene (2) eingreift.

5. Stent-Falt- und Ladevorrichtung nach einem der vorangehenden Ansprüche, **dadurch gekennzeichnet, dass** sich die Hohlschiene (2) in distaler Richtung über das distale Ende der Führungsschiene (4) hinaus erstreckt.

6. Stent-Falt- und Ladevorrichtung nach einem der vorangehenden Ansprüche, **dadurch gekennzeichnet, dass** eine sich parallel zu der Führungsschiene (4) erstreckende Aufnahmeschiene (14) vorhanden ist, welche ein distales freies Ende aufweist und derart ausgestaltet ist, dass ein Stent (36) vom distalen Ende her auf die Aufnahmeschiene (14) aufschiebbar ist.

7. Stent-Falt- und Ladevorrichtung nach Anspruch 6, **dadurch gekennzeichnet, dass** die Aufnahmeschiene von zwei sich seitlich des Eingriffssteges (4) erstreckenden Stäben (14) gebildet wird, wobei sich die beiden Stäbe beabstandet von dem Eingriffssteg (4) an entgegengesetzten Seiten des Eingriffssteges (4) erstrecken.

8. Stent-Falt- und Ladevorrichtung nach Anspruch 7, **dadurch gekennzeichnet, dass** ein Außenabstand der Stäbe (14) kleiner als ein Innendurchmesser eines aufzunehmenden Stents (36) ist.

9. Stent-Falt- und Ladevorrichtung nach einem der Ansprüche 6 bis 8, **dadurch gekennzeichnet, dass** die Aufnahmeschiene (14) mit ihrem distalen Ende elastisch relativ zu der Führungsschiene (4) in einer Richtung (Y) quer zur Längsachse (X) der Führungsschiene (4) auslenkbar ist.

10. Stent-Falt- und Ladevorrichtung nach einem der Ansprüche 6 bis 9, **dadurch gekennzeichnet, dass** sich die Aufnahmeschiene (14) mit ihrem distalen Ende distalwärts über das distale Ende (18) der Führungsschiene (4) hinaus erstreckt.

11. Stent-Falt- und Ladevorrichtung nach einem der vorangehenden Ansprüche, **dadurch gekennzeichnet, dass** der Innenquerschnitt der Hohlschiene (2) kleiner als der Außenquerschnitt eines aufzunehmenden Stents (36) ist.

12. Stent-Falt- und Ladevorrichtung nach einem der vorangehenden Ansprüche, **gekennzeichnet durch** einen axial entlang der Führungsschiene (4) vorschiebbaren Stößel (22).

13. Stent-Falt- und Ladevorrichtung nach Anspruch 12, **dadurch gekennzeichnet, dass** der Stößel (22) an seiner proximalen Seite zumindest eine Vorschubstange (28) aufweist, welche sich durch eine Führung (10) erstreckt, welche am proximalen Ende der Führungsschiene (4) angeordnet ist.

14. Bronchoskopsystem **gekennzeichnet durch** ein Bronchoskop (34), welches einen Bronchoskop-Tubus (32) und ein in diesem angeordnetes Endoskop (42) aufweist, sowie eine Stent-Falt- und Ladevorrichtung (1) nach einem der vorangehenden Ansprüche, wobei der Außenquerschnitt der Hohlschiene (2) und der Führungsschiene (4) in ihrer geschlossenen Position derart kleiner als der Innenquerschnitt des Bronchoskop-Tubus (32) ist, dass die Stent-Falt-und Ladevorrichtung (1) mit ihrem distalen Ende in eine distale Öffnung (43) des Bronchoskop-Tubus (32) einführbar ist.

15. Bronchoskopsystem nach Anspruch 14, **dadurch gekennzeichnet, dass** das Endoskop (42) in dem Bronchoskop-Tubus (32) exzentrisch angeordnet ist und die Führungsschiene (4) der Stent-Falt- und Ladevorrichtung (1), wenn diese in die distale Öffnung (43) des Bronchoskop-Tubus (32) eingeführt ist, dem distalen Ende des Endoskops (42, 44) gegenüberliegt oder sich radial einwärts parallel zu einem Schaft (44) des Endoskops (42) erstreckt.

16. Bronchoskopsystem nach Anspruch 14 oder 15 **gekennzeichnet durch** zusätzlich zumindest einen angepassten Stent (36), dessen Außendurchmesser größer als der Innendurchmesser des Bronchoskop-Tubus (32) und der Querschnitt der Hohlschiene der Stent-Falt- und Ladevorrichtung (1) ist und welcher gefaltet in der Hohlschiene (2) aufnehmbar ist.

## Claims

1. A stent folding and loading device (1) **characterised by**
a hollow rail (2) which has an arched cross section, as well as a guide rail (4) which forms an engagement web which faces the opening (8) of the hollow rail (2) and which extends in the longitudinal direction (X) of the guide rail (4), wherein
the hollow rail (2) at its proximal end is pivotably connected to a proximal end of the guide rail, and
the hollow rail (2) is pivotable relative to the guide rail (4), between a closed position, in which the longitudinal axes (X, X_{H}) of the guide rail (4) and the hollow rail (2) run in a parallel manner, and an opened position, in which the longitudinal axes (X, X_{H}) of the guide rail (4) and the hollow rail (2) run angled to one another.

2. A stent folding and loading device (1) according to claim 1, **characterised in that** the hollow rail (2) at its proximal end is pivotable relative to the guide rail (4) about a pivot axis (6), wherein the pivot axis (6) extends transversely to the longitudinal axis (X) of the guide rail (4) and to the longitudinal axis (X_{H}) of the hollow rail (2).

3. A stent folding and loading device according to claim 1 or 2, **characterised in that** the engagement web (4) in cross section extends in the diameter direction of the hollow rail (2).

4. A stent folding and loading device according to one of the preceding claims, **characterised in that** in the closed position, the engagement web (4) engages into the opening (8) of the hollow rail (2).

5. A stent folding and loading device according to one of the preceding claims, **characterised in that** the hollow rail (2) extends beyond the distal end of the guide rail (4) in the distal direction.

6. A stent folding and loading device according to one of the preceding claims, **characterised in that** a receiving rail (14) which extends parallel to the guide rail (4) is present, said receiving rail comprising a distal free end and being designed in a manner such that a stent (36) can be pushed onto the receiving rail (14) from the distal end.

7. A stent folding and loading device according to claim 6, **characterised in that** the receiving rail is formed by two rods (14) which extend laterally of the engagement web (4), wherein the two rods extend on opposed sides of the engagement web (4) in a manner distanced to the engagement web (4).

8. A stent folding and loading device according to claim 7, **characterised in that** an outer distancing of the rods (14) is smaller than an inner diameter of the stent (36) to be received.

9. A stent folding and loading device according to one of the claims 6 to 8, **characterised in that** the receiving rail (14) with its distal end can be elastically deflected relative to the guide rail (4) in a direction (Y) transverse to the longitudinal axis (X) of the guide rail (4).

10. A stent folding and loading device according to one of the claims 6 to 9, **characterised in that** the receiving rail (14) with its distal end extends distally beyond the distal end (18) of the guide rail (4).

11. A stent folding and loading device according to one of the preceding claims, **characterised in that** the inner cross section of the hollow rail (2) is smaller than the outer cross section of a stent (36) to be received.

12. A stent folding and loading device according to one of the preceding claims, **characterised by** a plunger (22) which can be axially advanced along the guide rail (4).

13. A stent folding and loading device according to claim 12, **characterised in that** the plunger (22) at its proximal side comprises at least one advance rod (28) which extends through a guide (10) which is arranged at the proximal end of the guide rail (4).

14. A bronchoscope system **characterised by** a bronchoscope (34) which comprises a bronchoscope tube (32) and an endoscope (42) which is arranged in this, as well as a stent folding and loading device (1) according to one of the preceding claims, wherein the outer cross section of the hollow rail (2) and of the guide rail (4) in their closed position is smaller than the inner cross section of the bronchoscope tube (32) to the extent that the stent folding and loading device (1) can be inserted with its distal end into a distal opening (43) of the bronchoscope tube (32).

15. A bronchoscope system according to claim 14, **characterised in that** the endoscope (42) is arranged eccentrically in the bronchoscope tube (32), and the guide rail (14) of the stent folding and loading device (1), when this is inserted into the distal end (43) of the bronchoscope tube (32), lies opposite the distal end of an endoscope (42, 44) or extends radially inwards parallel to a shank (44) of the endoscope (42).

16. A bronchoscope system according to claim 14 or 15, **characterised by** additionally at least one adapted stent (36) whose outer diameter is larger than the inner diameter of the bronchoscope tube (32) and the cross section of the hollow rail of the stent folding and loading device (1) and which in the folded condition can be received in the hollow rail (2).

## Revendications

1. Dispositif (1) de pliage et de mise en place d'une endoprothèse,
**caractérisé par**
un rail à gorge (2) qui présente une section transversale ayant la forme d'un arc, ainsi que par un rail de guidage (4) qui constitue une lame d'engagement orientée vers l'ouverture (8) du rail à gorge (2) et s'étendant dans la direction longitudinale (X) du rail de guidage (4), le rail à gorge (2) étant attaché de manière pivotante par son extrémité proximale à une extrémité proximale du rail de guidage,
et
le rail à gorge (2) étant pivotant par rapport au rail de guidage (4) entre une position fermée dans laquelle les axes longitudinaux (X, X_{H}) du rail de guidage (4) et du rail à gorge (2) s'étendent parallèlement et une position ouverte dans laquelle les axes longitudinaux (X, X_{H}) du rail de guidage (4) et du rail à gorge (2) s'étendent en enfermant un angle l'un par rapport à l'autre.

2. Dispositif (1) de pliage et de mise en place d'une endoprothèse selon la revendication 1, **caractérisé en ce que** le rail à gorge (2) est pivotant, à son extrémité proximale, autour d'un axe de pivotement (6) par rapport au rail de guidage (4), l'axe de pivotement (6) s'étendant transversalement à l'axe longitudinal (X) du rail de guidage (4) et à l'axe longitudinal (X_{H}) du rail à gorge (2).

3. Dispositif de pliage et de mise en place d'une endoprothèse selon la revendication 1 ou 2, **caractérisé en ce que** la lame d'engagement (4) s'étend, vu en coupe transversale, dans la direction du diamètre du rail à gorge (2).

4. Dispositif de pliage et de mise en place d'une endoprothèse selon l'une des revendications précédentes, **caractérisé en ce que**, en position fermée, la lame d'engagement (4) s'engage dans l'ouverture (8) du rail à gorge (2).

5. Dispositif de pliage et de mise en place d'une endoprothèse selon l'une des revendications précédentes, **caractérisé en ce que** le rail à gorge (2) s'étend dans la direction distale au-delà de l'extrémité distale de la lame d'engagement (4).

6. Dispositif de pliage et de mise en place d'une endoprothèse selon l'une des revendications précédentes, **caractérisé en ce qu'**il y a un rail de réception (14) s'étendant parallèlement au rail de guidage (4), qui comprend une extrémité libre distale et qui est conformé de façon telle qu'une endoprothèse (36) puisse être glissée sur le rail de réception (14) à partir de l'extrémité distale.

7. Dispositif de pliage et de mise en place d'une endoprothèse selon la revendication 6, **caractérisé en ce que** le rail de réception est constitué de deux tiges (14) s'étendant sur le côté de la lame d'engagement (4), les deux tiges s'étendant à une distance de la lame d'engagement (4) sur des côtés opposés de la lame d'engagement (4).

8. Dispositif de pliage et de mise en place d'une endoprothèse selon la revendication 7, **caractérisé en ce qu'**une distance extérieure des tiges (14) est inférieure à un diamètre intérieur d'une endoprothèse (36) à recevoir.

9. Dispositif de pliage et de mise en place d'une endoprothèse selon l'une des revendications 6 à 8, **caractérisé en ce que** le rail de réception (14) est susceptible d'être dévié élastiquement à son extrémité distale, par rapport au rail de guidage (4), dans une direction (Y) transversalement à l'axe longitudinal (X) du rail de guidage (4).

10. Dispositif de pliage et de mise en place d'une endoprothèse selon l'une des revendications 6 à 9, **caractérisé en ce que** le rail de réception (14) s'étend, par son extrémité distale, dans la direction distale au-delà de l'extrémité distale (18) du rail de guidage (4).

11. Dispositif de pliage et de mise en place d'une endoprothèse selon l'une des revendications précédentes, **caractérisé en ce que** la section transversale intérieure du rail à gorge (2) est inférieure à la section transversale extérieure d'une endoprothèse (36) à recevoir.

12. Dispositif de pliage et de mise en place d'une endoprothèse selon l'une des revendications précédentes, **caractérisé par** un poussoir (22) adapté pour pouvoir être avancé axialement le long du rail de guidage (4).

13. Dispositif de pliage et de mise en place d'une endoprothèse selon la revendication 12, **caractérisé en ce que** le poussoir (22) comprend à son côté proximal au moins une tige d'avancement (28) qui s'étend à travers un guidage (10) qui est disposé à l'extrémité proximale du rail de guidage (4).

14. Système de bronchoscope **caractérisé par** un bronchoscope (34) qui comprend un tube de bronchoscope (32) et un endoscope (42) disposé dans celui-ci, ainsi qu'un dispositif (1) de pliage et de mise en place d'une endoprothèse selon l'une des revendications précédentes, la section transversale extérieure du rail à gorge (2) et du rail de guidage (4) en leur position fermée étant inférieure à la section transversale intérieure du tube de bronchoscope (32) d'une manière telle que le dispositif (1) de pliage et de mise en place d'une endoprothèse soit susceptible d'être introduit, par son extrémité distale, dans une ouverture distale (43) du tube de bronchoscope (32).

15. Système de bronchoscope selon la revendication 14, **caractérisé en ce que** l'endoscope (42) est disposé dans le tube de bronchoscope (32) de façon excentrique et que le rail de guidage (4) du dispositif (1) de pliage et de mise en place d'une endoprothèse, lorsque celui-ci est introduit dans l'ouverture distale (43) du tube de bronchoscope (32), est opposé à l'extrémité distale de l'endoscope (42, 44) ou s'étend, radialement vers l'intérieur, parallèlement à une tige (44) de l'endoscope (42).

16. Système de bronchoscope selon la revendication 14 ou 15, **caractérisé par** en plus au moins une endoprothèse (36) adaptée dont le diamètre extérieur est supérieur au diamètre intérieur du tube de bronchoscope (32) et à la section transversale du rail à gorge du dispositif (1) de pliage et de mise en place d'une endoprothèse, et qui est susceptible d'être reçu dans le rail à gorge (2) en état plié.
